(19) Europäisches Patentamt

European Patent Office

Office européen des brevets

(11) **EP 0 416 150 B1**

(12) **EUROPÄISCHE PATENTSCHRIFT**

(45) Veröffentlichungstag und Bekanntmachung des Hinweises auf die Patenterteilung:
**14.05.1997 Patentblatt 1997/20**

(51) Int. Cl.$^6$: **A61N 5/06**, A61H 39/00

(21) Anmeldenummer: 89116523.5

(22) Anmeldetag: **07.09.1989**

(54) **Subminiatur-Therapie-Lasergerät zur Biostimulation von organischem Gewebe**

Subminiature therapeutic laser apparatus for the biostimulation of organic tissue

Appareil sous-miniature pour thérapie à laser pour biostimulation de tissu organique

(84) Benannte Vertragsstaaten:
**AT BE CH DE ES FR GB IT LI NL SE**

(43) Veröffentlichungstag der Anmeldung:
**13.03.1991 Patentblatt 1991/11**

(73) Patentinhaber: **Oppold, Eberhard, Dipl.-Ing.**
**61250 Usingen (DE)**

(72) Erfinder: **Oppold, Eberhard, Dipl.-Ing.**
**61250 Usingen (DE)**

(56) Entgegenhaltungen:
EP-A- 0 272 325        EP-A- 0 298 138
DE-A- 3 226 507

Bemerkungen:
Die Akte enthält technische Angaben, die nach dem Eingang der Anmeldung eingereicht wurden und die nicht in dieser Patentschrift enthalten sind.

## Beschreibung

Die vorliegende Erfindung betrifft ein Subminiatur-Laser-Gerät zur therapeutischen Bestrahlung von organischem Gewebe.

Die vorgegebene minimale Größenordnung der Gehäuseabmessungen soll es trotzdem ermöglichen, ein Therapielasergerät im Leistungsbereich von 0,5 bis 10 mW mit ausreichenden Sicherheitsvorkehrungen, relativ hoher Leistungsdichte, verstellbarer Strahlfokussierung und einer Funktionskontrolle als ständigen ungefährlichen "Begleiter" für den Benutzer zu beinhalten.

Stand der Technik

Ähnliche Geräte sind in größerer Ausführung als sogenannte Mid- oder Soft-Laser in der Medizin bekannt. Sie sind üblicherweise als netzgespeiste Stand- oder Tischgeräte ausgebildet und werden im Rahmen einer medizinischen Praxis im ambulanten, bzw. stationären Bereich eingesetzt. Ihr bioregulatorischer Effekt ist vielfach bewiesen. Unter Prof. Endre Mester wurden seit 1967 Versuche durchgeführt, die das in zunehmendem Maß weitgehend bestätigten. Eine Zusammenfassung der Versuche findet man zum Beispiel in der Arbeit von Prof. E. Mester: Laser Application in Promoting of Wound-Healing", die in "Laser in Medicine", Ausgabe 1980, veröffentlicht ist (Herausgeber: H.K. Koebner, Wiley-Interscience Publ. 1980). Eine weitere Zusammenfassung der Versuche von Prof. Mester "Der Laser" wurde herausgegeben von K. Dinstlund P.L. Fischer, Springer Verlag 1981.

Dr. G. Lonauer weist in der Zeitschrift "Die Medizinische Welt" 29/85 in dem Artikel "Konservative Lasertherapie auf die Erfolge von Dr. Ludtmann bei der Bestrahlung von Herpes-simplex-Infektionen mit MID - Laser - Bestrahlung hin.

Frau Dr. J. Hachenberger-Wildner berichtet in der Zeitschrift "Ärztliche Kosmetologie", Heft 2, März/April 1981, S. 142-144, "Laserstrahlen bei Herpes-Erkrankungen" über Erfahrungen bei der Bestrahlung mit 632,8 nm (He-Ne) Laser. Sie weist auf folgendes hin: " Die praktische Durchführung der Bestrahlung ist abhängig von der Leistung des Lasers. Hiernach richten sich Bestrahlungsabstand und -zeit."

Zu Herpes simplex: "Unabhängig von der Größe und der Lokalisation des Herdes waren in der Regel 1 bis 3 Bestrahlungen erforderlich. Die Abheilung konnte damit innerhalb 1 bis 3 Tagen erreicht werden. Je früher die Patienten zur Behandlung kamen desto schneller war der Erfolg zu verzeichnen."

Dr. Josef Bahn weist in seinem Buch "Laser- und Infrarotstrahlung in der Akupunktur" (Haug 1987) auf Seite 36 zuletzt darauf hin, daß in manchen Fällen bei Herpeserkrankungen doch mehr Behandlungen - oft 7 bis 10- erforderlich sind.

Dr. Ulrich Warnke (Universität des Saarlandes, Saarbrücken, Fachbereich 16.4 Biologie) trifft in der Veröffentlichung "Der Dioden Laser" die kalkulierte Feststellung für gepulste Diodenlaser um 900 nm: Intakte Zellen werden von der Laserstrahlung im Ergebnis keine Änderung erfahren, während"kranke"Zellen energetisch aufgebaut werden können. Dt. Ärzteblatt 84, Heft 44, 29.10.87.

Bei Geräten mit Laserstrahlaufweitung wird ein Dauerstrich-Laser mit 633 nm He-Ne beschrieben, der mit einem 840 nm IR-cw-Laser überlagert ist, dessen Strahl bis 30 mm aufgeweitet wird, um das zu bestrahlende Feld in der Fläche variabel zu gestalten (Offenlegungsschrift v.6.8.87 DE 3603156 A1 ; A 61N 5/06).

In der Patentschrift DE 2548354 C2 (A 61 N 5/06) wird ein begründet nicht fokussierter 2 mW cw-Laser mit elektromechanischer Vorrichtung zur Modulierung des Laserstrahles im Bereich von 2 bis 20 Hz dargestellt, der besonders für die Laser-Akupunktur konzipiert wurde. Auf Seite 2 (9-20) wird auf den vermeintlichen Vorteil eines größeren Strahldurchmessers bei Bestrahlung der Akupunkturpunkte hingewiesen.

Im Lehrbuch von Berlien und Müller "Angewandte Lasermedizin", herausgegeben vom Laser-Medizin-Zentrum Berlin 1989, erschienen im Ecomed Verlag mbH, Landsberg-München-Zürich weist Dr.med.vet. J.H. Walter unter II-3.1 "Eigenschaften von biologischen Geweben", Seite 2, auf folgendes hin: Bedeutsamkeit der Laserstrahl-Streuung im biologischen Gewebe, besonders im Bereich der Wellenlänge von 590 nm bis 1500 nm. Es wird angeführt, daß im biologischen Gewebe dieser Wellenlängen die kollimierte Strahlqualität verloren geht und durch einen diffusen Streukegel ersetzt wird.

Im gleichen Lehrbuch weist Dr.rer.nat. K. Dörschel, Berlin unter II - 2.3 auf Seite 3 darauf hin, daß die Leistungsdichte und die Einwirkzeit die hauptsächlichen Parameter eines Laserstrahls sind, die die Wirkung auf Gewebe beeinflussen. Dabei gilt

$$\text{Leistungsdichte} = \frac{\text{Laserstrahlleistung}}{\text{Strahlquerschnitt}}$$

Zur technischen Spezifikation der einzelnen Lasereinrichtungen zur Biostimulation schreibt Dr.rer.nat., Dipl.-Ing. R. Senz, Berlin, in "Angewandte Lasermedizin" unter II - 3.2, Seite 2: "Bis heute ist nicht bekannt, welche Wellenlänge die am besten geeignete für biochemische Reaktionen im Bereich der Biostimulation ist. Wellenlängen im Bereich von 442-1064 nm sind hierzu untersucht worden." Aus dem Bereich der praktischen Anwendung gibt es viele Studien mit zum Teil kontroversen Ergebnissen. Es werden Laserlicht - Behandlungen mit Helium-Neon (633 nm) oder Gallium - Arsenid (904 nm) oder eine Kombination aus beiden beschrieben. Einige benutzen Dauerstrichlaser (cw), andere jedoch gepulste oder modulierte Systeme. Senz schreibt hierzu auf Seite 3: "Es gibt jedoch keine systematischen Studien, die eine Korrelation zwischen Wellenlänge und biologischem Effekt aufzeigen. Weiterhin werden recht verschiedene Energiedichten am Gewebe eingesetzt. Dies erklärt auch die recht unterschiedlichen Ergebnisse der verschiedenen Arbeitsgruppen."

Die internationale Anmeldung PCT/JP86/00666 mit der EP-Veröffentlichungsnummer 0272 325 beschreibt ein aufwendiges Halbleiter-Laser-Therapiegerät, bei dem aus einer Mehrzahl von verschiedenen Laserdioden, hierbei 3 Stück, über ein justierbares optisches System ein vor der Gehäuseöffnung fokussiertes Strahlenbündel erzeugt wird.

Das Gerät hat einen mechanischen Berührungsschalter, der bei Berührung mit dem zu behandelnden menschlichen Körper seinen Schaltzustand ändert. Diese Schaltsignale werden in einem Schaltkreis zusammen mit anderen Signalen zur Ermittlung und Steuerung der Bestrahlungszeit verwendet.

Auf dem Weg zur Miniaturisierung sind sogenannte Pocket-Laser mit einem Gehäusevolumen von über 65 cm$^3$ und einer Gesamtlänge von z.Teil mehr als 14 cm bekannt, die mit 633nm (He-Ne) oder 670 nm (InGaAlP Dioden) hauptsächlich als Pointer, Richtlaser, Positionierlaser und Pilotstrahl Verwendung finden. Da sie eine feststehende Kollimatoroptik mit nahezu parallelem Laser -Ausgangsstrahl großer Reichweite aufweisen, ist bei ihrem Betrieb eine Gefährdung des menschlichen und tierischen Auges bei optischen Ausgangsleistungen von $\geqq$ 1mW im cw - Betrieb nicht auszuschließen.

In der Offenlegung DE 3226507 A1 wird ein batterie- oder akkubetriebenes lichttechnisches Gerät für akupunkturähnliche therapeutische Behandlung vorgestellt, bei dem verschiedene optische und/oder mechanische Einsätze auf die Laserdiode aufgesteckt werden können, um den austretenden Strahlcharakter anwendungsdienlich zu verändern. Der Abstand zwischen Strahlaustrittsöffnung und der zu bestrahlenden Fläche kann während der Bestrahlung variabel sein und bedingt somit veränderliche Leistungsdichten während der Bestrahlung, ausgenommen der Strahl wird parallel ausgerichtet oder die Laserdiode wird direkt auf die zu bestrahlende Fläche aufgesetzt, wobei eine geringere Leistungsdichte auf der zu bestrahlenden Fläche hingenommen werden muß.

## Kritik am Stand der Technik

Mid- oder Soft-Lasergeräte, die in der Medizin Anwendung finden, sind durch ihre Baugröße bedingt als ständige"Begleiter" in der Hosen-, Hemden- oder Handtasche ungeeignet, obgleich es für den Bereich der Eigentherapie, wie z.B. bei Herpes simplex - Erkrankungen, für die Laser-Akupunktur, die akute Schmerzbekämpfung und bei ambulanter Biostimulation wünschenswert ist.
Die vorhandenen sogenannten Pocket - Laser sind wegen ihrer zu geringen Leistungsdichte (z.B. 1mW bei 4mm festem Austrittsstrahldurchmesser) und mangelhafter, bzw. fehlender Sicherheitsvorkehrungen als Therapielaser ungeeignet. Außerdem sind sie noch zu groß, um sie in einer Hemden- oder Hosentasche problemlos wie ein Feuerzeug tragen zu können und somit immer zur Verfügung zu haben.

## Aufgabe

Hieraus ergibt sich die Aufgabe, ein batterie- oder akkubetriebenes miniaturisiertes Therapie-Lasergerät zu konstruieren, das mit kostengünstigen cw-Laserdioden kleiner und mittlerer Leistung auf den zu behandelnden Oberflächen von organ. Gewebe die zur Biostimulation und Therapie erforderlichen Leistungsdichten durch die entsprechende Aufbereitung des Laserstrahls erreicht. Zugleich muß das Gerät die für seinen Leistungsbereich erforderlichen technischen Sicherheits- und Kontrolleinrichtungen besitzen, die die Augen des Benutzers und sein gefährdetes Umfeld vor einem Laserstrahl mit hoher Leistungsdichte schützen und zugleich eine sichere und leicht überprüfbare Funktion des Gerätes garantieren.

## Lösung

Die Aufgabe wird bei einer gattungsgemäßen Einrichtung durch die Merkmale des Anspruchs 1 gelöst. Zwei Ausführungsbeispiele der Erfindung sind nachfolgend anhand der Zeichnungen beschrieben.
Es zeigen:

Fig.1 Schematisch vereinfacht dargestelltes Subminiatur-Therapie-Lasergerät im Maßstab 2 : 1 mit integriertem Gleichspannungswandler.

Fig.2 Schematisch vereinfacht dargestelltes Subminiatur-Therapie-Lasergerät im Maßstab 2 : 1 in Seitenansicht A für den Betrieb mit 3 Micro-Batterien und höherer Laserdioden-Ausgangsleistung.

Fig.3 Seitenansicht B der Darstellung von Fig .2

Fig.4 Subminiatur-Therapie-Lasergerät in Originalgröße mit dem mobilen Laserleistungs- und Modulationsmeßgerät, schematisch sehr vereinfacht dargestellt.

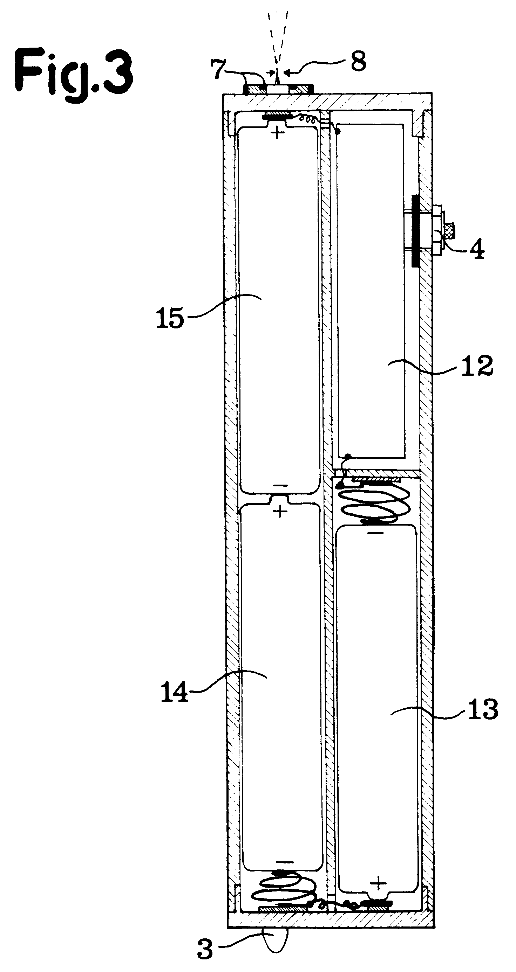

Um ein solches Subminiatur-Therapie-Lasergerät bis zu einer Leistung von ca. 5mW zu fertigen, wird entsprechend dem Beispiel von Fig .1 (Maßstab 2 : 1) mit einer Spannungsquelle (6) von 1 bis 1,6 Volt gearbeitet, die hier als Mignonzelle ausgebildet ist. Die für die Speisung der cw - Laserdiode erforderliche Spannung wird mit Hilfe eines integrierten Schaltkreises in SMD - Bauweise bereitgestellt, der 5 Volt Ausgangsspannung liefert und einen Teil der Steuerelektronik (5) darstellt. Entsprechend dem Unteranspruch 3 ist es für höhere opt. Ausgangsleistung bei der Verwendung von cw-Dioden bis ca. 10 mW Laserausgangsleistung sinnvoll, mit drei Aikaline-Batterien oder Ni-Cad-Sinterzellen Fig.3 (13, 14, 15) geringer Baugröße zu arbeiten, da der Miniaturisierung von Gleichspannungswandlern bei größerem Strombedarf und gefordert hohem Wirkungsgrad durch physikalische Gesetzmäßigkeiten und zur Zeit verfügbare Bauteile deutlich Grenzen gesetzt sind.

Um das Gerät für den Benutzer und sein Umfeld technisch sicher zu gestalten, ist erfindungsgemäß eine elektronische Einschaltsperre so angeordnet, daß ein Einschalten des Gerätes mit dem Druckknopfschalter (4) nur dann möglich ist, wenn zugleich der Sensorring (7) mit der Haut des Patienten oder Benutzers Kontakt hat. Die dazu erforderliche Elektronik ist in (5) oder (12) angeordnet.

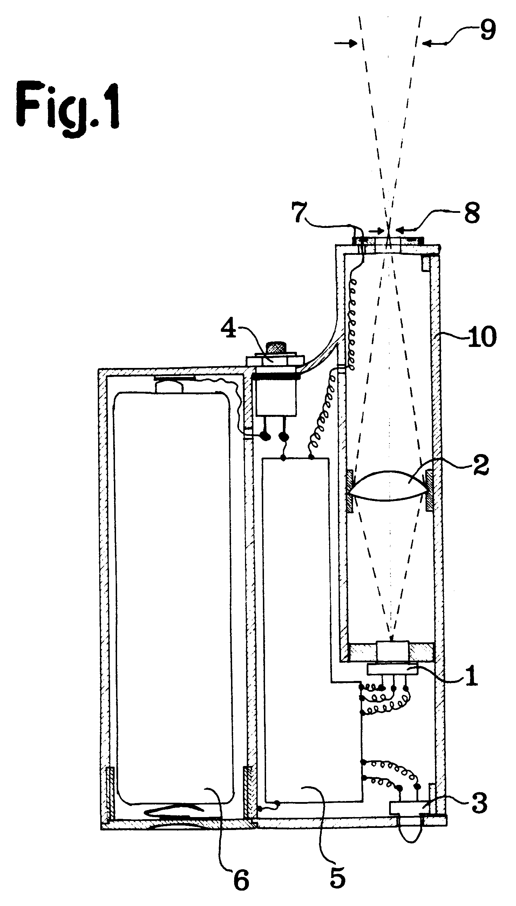

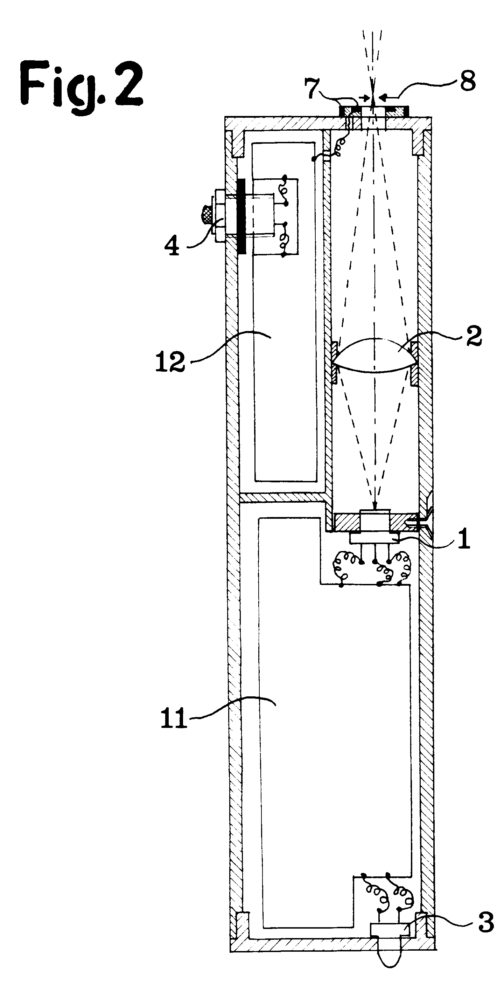

Da sich der Sensorring (7) entsprechend Fig. 1, 2, 3 in der unmittelbaren Nähe der höchstmöglichen Laserstrahl-Leistungsdichte (8) befindet, ist eine Gefährdung für den Benutzer des Gerätes und sein Umfeld in Zusammenhang mit dem Gesamtverlauf des Laserstrahles Fig.1 (8, 9) bei normalem Gebrauch und dem vorgegebenen Leistungsbereich unmöglich.

Nach Unteranspruch 4 wird eine low-power Leuchtdiode, meist mit einem Betriebsstrom von $\leq$ 1mA (3) zur erforderlichen Anzeige des Betriebszustandes des Gerätes eingesetzt. Die Eigentherapie von Herpes simplex (labialis) sollte hierbei zweckmäßig vor einem Spiegel erfolgen, 1S um die Kontrolldiode (3) beobachten zu können.

Dieser Nachteil kann durch einen zusätzlichen akustischen Signalgeber geringer Baugröße ausgeglichen werden. Eventuell kann dann auf die optische Anzeige verzichtet werden, so man die Geräuschbelästigung zu tolerieren bereit ist.

Die für die Therapie, bzw. Stimulation von organischem Gewebe entscheidende Leistungsdichte wird erfindungsgemäß erreicht mit Hilfe einer veränderlichen Fokussiereinrichtung (2), unter Berücksichtigung der jeweils eingesetzten Laserdiode (1).

Die höchste Leistungsdichte auf dem zu behandelnden Gewebe, bei vorgegebener Laserdiode, wird erreicht wenn sich der Fokus des Laserstrahles (8) unmittelbar vor der Strahlaustrittsöffnung befindet. Beim Einsatz von Laserdioden, die eine größere optische Ausgangsleistung (>3 mW) haben, kann es erforderlich werden, den einstellbaren Fokus in den Bereich des Gehäuses zurückzunehmen. Dies hat zur Folge, daß der Laserstrahl an der Austrittsöffnung im Bereich des Sensorringes (7) einen größeren Durchmesser aufweist bei zugleich reduzierter Leistungsdichte. Bei konstanter Gewebeoberflächengröße bedeutet dies eine verkürzte Bestrahlungsdauer.

Eine Verstellmöglichkeit des Fokus im Strahlenverlauf des Lasers wird auch in Anlehnung an die landesunterschiedlichen gesetzlichen Anforderungen geschehen. Zum Beispiel durch ein Verschieben der Optik (2) im Gehäuse, nachdem die Gehäuseabdeckung Fig.1 (10) geöffnet wurde, oder durch entsprechende Stellschrauben, die von außen zugänglich sind. Natürlich kann der gleiche Effekt durch entsprechende Positionsveränderung der Laserdiode (1) erreicht werden.

Entscheidend für die Sicherheit ist ein vorgegebener mechanischer Anschlag im Bereich der Verstelleinrichtung, der es verhindert, daß der Fokus des Laserstrahles im Verlauf weiter vor die Austrittsöffnung verlegt werden kann.

Für den speziellen Akupunktur - Anwendungsbereich wird das Subminiatur-Therapie-Lasergerät entsprechend dem Unteranspruch 5 mit einem in der Frequenz einstellbaren Modulator für den Bereich 0,5 bis 30 Hz ausgerüstet.

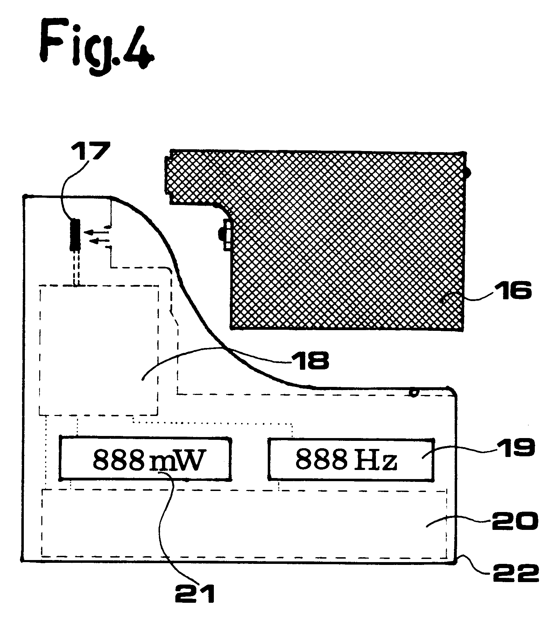

Die Frequenz wird mit Hilfe eines passenden Leistungs- und Modulationsmeßgerätes Fig.4 (22) genau eingestellt (19). Dazu befindet sich in Höhe der Steuerelektronik (5, 11) ein Trimmpotentiometer, der von außen mit einem Schraubendreher verstellt werden kann. Die Modulation kann über einen Microswitch von außen zu-, bzw. abgeschaltet werden.

Die gesamte erforderliche Steuerelektronik ist entsprechend dem zur Verfügung stehenden Raumangebot vorzüglich in SMD-Technik gefertigt und in den Bereichen (5), (11) und (12) angeordnet.

Um die abgestrahlte Laserleistung genau bestimmen zu können, wird entsprechend dem Unteranspruch 6 ein Laserleistungs- und Modulationsmeßgerät Fig.4 (22) eingesetzt, das entsprechend seiner Formgebung das Subminiatur-Therapie-Lasergerät so aufnehmen kann, daß es dies in Betrieb setzen kann und die abgestrahlte Laserleistung und ggf. Modulation anzeigt.

Das unter Fig. 4 dargestellte System enthält das Subminiatur-Therapie-Lasergerät (16) in seiner Originalgröße und das Laserleistungs- und Modulationsmeßgerät (22). Das Meßgerät (22) sollte eine Anzeige im Bereich von mW (21) haben, um die abgegebene Laserleistung zu messen sowei eine getrennte Anzeige in Hz (19) für die genaue Einstellung und Anzeige der Laserstrahlmodulation.

Die von dem optischen Sensor (17) erfaßte Laserstrahlung wird über eine Auswertungs- und Treiberstufe (18) den Anzeigeinstrumenten (19, 21) zugeleitet. Die Stromversorgung (20) befindet sich im Gerät.

Erzielbare Vorteile

Die mit der Erfindung erzielbaren Vorteile bestehen insbesondere darin, daß das beschriebene Subminiatur-Therapie-Lasergerät die für die Soft-Laser-Therapie, bzw. Biostimulation von organischem Gewebe erforderliche Leistungsdichte mit billigen, aus der Massenfertigung stammenden cw - Laserdioden mit geringer Ausgangsleistung, erreicht.

Bedingt durch die bei dieser Erfindung erreichte Baugröße, wird ein Therapie-Lasergerät hergestellt, das in jeder Hemden- und Hosentasche problemlos wie ein Feuerzeug zu tragen ist und somit immer und fast überall dem Anwender zur Verfügung stehen kann.

Die Einsatzmöglichkeit entspricht allen möglichen Anwendungsgebieten der herkömmlichen Großgeräte. Bauartbedingt ist das Gerät nur für wenige Applikationen der Soft- oder Mid-Laser-Therapie ungeeignet, wie bei der Behandlung von Ulcera, frischen Wunden, Anwendungen im dental-medizinischen Bereich und überall dort, wo bei großflächiger Anwendung geringe Bestrahlungszeit gefordert wird.

Für den Anwendungsbereich von Herpes simplex (labialis) kann die erforderliche Therapiedauer durch einen verzögerten Behandlungsbeginn sehr stark verlängert werden. Bei bereits gut sichtbaren Lippenbläschen des Patienten können bis zum Abheilen mit täglicher Soft-Laser-Behandlung 2 bis 5 Tage vergehen. Erfolgt hingegen eine Laser-Behandlung mit dem Subminiatur-Therapie-Laser 780 nm und 3mW von 2 bis 4 Minuten Dauer (je nach Größe der befallenen labialen Stelle), unmittelbar nachdem der Patient den akuten Herpes verspürt, so reicht in der Regel eine einmalige Bestrahlung und es kommt nicht zum Ausbruch der labialen Bläschen.

Für die Laser-Akupunktur wird das Gerät entsprechend Unteranspruch 5 mit einem Modulator ausgerüstet, so daß unter Berücksichtigung des Unteranspruchs 8 mit einstellbaren Modulationsfrequenzen nach Nogier gearbeitet werden kann.

Ein sicherer Funktions- und Leistungstest des Subminiatur-Therapie-Lasergerätes ist durch die Formgebung des Gehäuses in Zusammenhang mit der Ausgestaltung des Testgerätes entsprechend dem Beispiel in Fig.4 gewährleistet, so daß bei dem Meßvorgang keinerlei Streustrahlung austreten kann. Zugleich wird die genaue Einstellung einer Modulationsfrequenz ermöglicht, ohne das eigentliche Therapiegerät in seiner Konstruktion mit dieser Einrichtung zu belasten.

Entsprechend den verfügbaren Laserdioden kann das Gerät für verschiedene Wellenlängen des Laserlichtes hergestellt werden, bevorzugt jedoch für 780 nm oder 670 nm, da diese Bereiche im therapeutisch wirksamen Spektrum sind und zugleich für das Auge noch sichtbar und somit höhere Sicherheit bieten.

Das erfindungsgemäße ausgebildete Gerät weist für seine Baugröße ein Höchstmaß an autonomer Sicherheit auf, bedingt durch die große Strahldivergenz im weiteren Verlauf des Laserstrahles außerhalb des Gerätes sowie des zusätzlichen Sensorschalters im unmittelbaren Strahlaustrittsbereich.

Das Gerät ist somit im humanmedizinischen Bereich vorzüglich zur Eigentherapie geeignet und stellt im Bereich der Tiermedizin für den Anwender eine wesentliche Erleichterung in der Handhabung dar.

Bedingt durch eine einfach mögliche industrielle Massenfertigung dieses Subminiatur-Therapie-Lasergerätes, bei den zum Einsatz kommenden preiswerten Komponenten, eignet sich das Gerät zugleich als kostengünstiges Soft-Laser-Gerät für jeden Anwender.

**Patentansprüche**

1. Subminiatur-Therapie-Lasergerät zur Biostimulation von organischem Gewebe, insbesondere zur Eigentherapie für die Behandlung von Herpes simplex, zur Laser Akupunktur und zur Schmerzbekämpfung, umfassend ein Gehäuse mit einem Gehäusevolumen von circa 50 bis 65 cm3, mit darin angeordneten Elementen umfassend eine Spannungsquelle (6), eine Halbleiter Dauerstrich-Laser-Diode (1), eine Laserstrahlfokussierung (2), sowie eine Einschaltsperre, wobei die Laser-Diode (1) Licht im sichtbaren roten bis nahen IR-Bereich mit einer Ausgangsleistung von 0.5 bis 10 mW emittiert, wobei die Laserstrahlfokussierung eine Optik (2) umfaßt, wobei die Einschaltsperre über einen Sensorring (7), der um die Laserstrahlaustrittsöffnung angeordnet ist, bei Kontakt mit von zu bestrahlendem Gewebe ausgeschaltet wird, und wobei die Laserdiode (1) und die Optik (2) so zueinander positioniert werden können, daß der Strahlfokus in einem Bereich von unmittelbar vor dem Gehäuse bis, laserquellenwärts, 1 cm hinter die Laseraustrittsstelle innerhalb des Gehäuses zu liegen kommt, so daß auf das an dem Sensorring anliegende Gewebe eine fest einstellbare Leistungsdichte einwirken kann und zugleich, bedingt durch die Divergenz und der damit verbundenen verringerten Leistungsdichte im weiteren Strahlverlauf (9), geringe Gefahr für das menschliche Auge besteht.

2. Subminiatur-Therapie-Lasergerät nach Anspruch 1, dadurch gekennzeichnet, daß das Gerät bei einer optischen Ausgangsleistung bis 5 mW nur eine Betriebsspannung von 1 bis 1,6 Volt benötigt, bedingt durch einen integrierten miniaturisierten Gleichspannungswandler.

3. Subminiatur-Therapie-Lasergerät nach Anspruch 1, dadurch gekennzeichnet, daß das Gerät für eine höhere optische Ausgangsleistung bis zu 10 mW auch mit 3 x 1,5 Volt Micro Alkaline Batterien oder vergleichbaren Akkus ausgerüstet sein kann (13, 14, 15).

4. Subminiatur-Therapie-Lasergerät nach Anspruch 1, dadurch gekennzeichnet, daß eine Anzeige des Betriebszustandes des Gerätes durch eine low-power Leuchtdiode (3) und/oder einen akustischen Signalgeber geringer Baugröße gegeben ist.

5. Subminiatur-Therapie-Lasergerät nach Anspruch 1, dadurch gekennzeichnet, daß die Ansteuerelektronik der Laserdiode (5, 11 ) für den speziellen Akupunktur - Anwendungsbereich einen frequenzvariablen Modulator für den Bereich von 0.5 bis 30 Hz enthalten kann.

6. Subminiatur-Therapie-Lasergerät nach Anspruch 1, dadurch gekennzeichnet, daß die Formgebung des Gehäuses einen Einschub in ein mobiles Laser-Leistungs- und Modulationsmeßgerät erlaubt und daß beim Einsetzen des Therapie-Lasergerätes (16) in das mobile Laser-Leistungs- und Modulationsmeßgerät (22) der Schalter (4) des Therapie-Lasergerätes (16) von diesem betätigt wird durch die einander angepaßte Formgebung der Gehäuse von Laser-Leistungs- und Modulationsmeßgerät (22) zu dem Therapie-Lasergerät (16) und dieses einschaltet, wobei eine optische Abschirmung des Laser-Leistungs- und Modulationsmeßgerätes (22) im Bereich des Überganges von der Laserstrahlaustrittsstelle aus dem Gehäuse des Lasergerätes (16) zu dem optischen Sensor (17) gefährliche Streustrahlung während des Meß- und Einstellvorganges am Austritt hindert.

## Claims

1. Subminiature therapeutic laser apparatus for the biostimulation of organic tissue, especially for the self-treatment of herpes simplex, for laser acupuncture and for the alleviation of pain,
comprising a casing having a volume of about 50 to 65 cm3 including elements located in said casing comprising a supply point (6), a semiconductor continous-wave laser diode (1), a laser beam focusing (2) as well as a lock-out device, said laser diode emitting light in the visible red to the near-infrared region with a power output of 0.5 up to 10 mW, said laser beam focusing comprising a lens (2), said lock-out device being switched off, when it comes into contact with tissue to be irradiated, by means of a sensor ring located around the laser beam emergence opening,
said laser diode (1) and the lens (2) being positioned in a way that the beam focus lies in the region from directly in front of the casing to, towards the laser source, 1 centimetre behind the laser emergence opening within the casing, so that an adjustable power density can act on the tissue adjacent to the sensor ring, and at the same time there's little danger for the human eye due to the divergence and, in consequence, the reduced power density in the further course of the radiation (9).

2. An apparatus according to Claim 1, characterized in that said apparatus, having an optical output of up to 5 mW, only needs a line voltage of 1 to 1.6 V, due to an integrated miniaturized d.c. converter.

3. An apparatus according to Claim 1, characterized in that said apparatus can also be equiped with three Micro Alkaline batteries at 1.5 V or comparable accumulators in order to reach a higher optical output of up to 10 mW (13, 14, 15). The apparatus according to invention shows a maximum amount of autonomous safety in relation with its small physical size, due to the big beam divergence in the further course of the laser beam outside said apparatus and due to the additional sensor switch which is directly in the beam emergence region.
Therefore said apparatus is especially appopriate for the self-treatment in the field of human medicine; it allows the user a considerably easier handling in the field of veterinary medicine.
As industrial mass production is easily possible and the used components are cheap, said subminiature therapeutic laser apparatus is suitable at the same time for every user as an economical soft laser apparatus.

4. An apparatus according to Claim 1, characterized in that a low power luminous diode (3) and/or an acustic signal transmitter of small size indicate the working condition of the apparatus.

5. An apparatus according to Claim 1, characterized in that the drive electronics of the laser diode (5, 11) can comprise a modulator with variable frequency in the range from 0.5 to 30 Hz for the special acupuncture application.

6. An apparatus according to Claim 1, characterized in that the shape of its casing allows the insertion into a mobile laser modulation and power meter, and in that when said therapeutic laser apparatus (16) is inserted into the mobile laser modulation and power meter (22), the switch (4) of said therapeutic laser apparatus(16) is activated by the mobile laser modulation and power meter (22), due to the casing shapes of the laser modulation and power meter (22) and the therapeutic laser apparatus (16), which are adapted to each other, and said therapeutic laser apparatus starts working, an optical shielding of the laser modulation and power meter (22) preventing dangerous leakage radiation from emerging during the measurement and adjustment process in the region of transition from the laser-beam emergence point out of the casing of the laser apparatus (16) to the optical sensor (17).

## Revendications

1. Appareil sous-miniature pour thérapie à laser pour biostimulation de tissu organique, particulièrement pour l'auto-thérapie de l'herpès simplex, pour l'acuponcture laser et pour l'apaisement de la douleur,
comportant un boîtier d'un volume d'environ 50 à 65 cm3 contenant des éléments comportant une source de tension (6), une diode laser à semi-conducteurs à onde continue (1), une focalisation du rayon laser (2) et un dispositif qui bloque la fermeture, ladite diode laser (1) émettant la lumière dans le domaine visible des rayons rouges jusqu'au domaine des rayons infrarouges proches avec une puissance de sortie de 0,5 à 10 mW,
ladite focalisation du rayon laser comportant un objectif (2),

ledit dispositif qui bloque la fermeture étant interrompu, s'il entre en contact avec du tissu à traiter aux rayons, à l'aide d'un senseur (7) autour de l'orifice de sortie du rayon laser,

et ladite diode laser et l'objectif étant arrangés de manière que le foyer du rayon se trouve dans un domaine d'immédiatement devant le boîtier jusqu'à 1 cm, en direction de la source laser, derrière la sortie du rayon laser dans le boîtier de sorte qu'une densité de puissance règlable peut agir sur le tissu contigu au senseur annulaire, et en même temps, le traitement ne présente qu'un risque minime pour l'oeuil, dû à la divergence et en conséquence à la densité de puissance réduite dans la marche des rayons.

2. Appareil suivant la revendication 1, caractérisé par le fait que l'appareil, ayant une puissance optique de sortie jusqu'à 5 mW, ne nécessite qu'une tension de fonctionnement de 1 à 1,6 V, dû à un convertisseur continu-continu integré miniaturisé.

3. Appareil suivant la revendication 1, caractérisé par le fait que l'appareil, pour une puissance optique élevée de sortie de 10 mW au maximum, peut être équipé de trois battéries Mikro Alkaline à 1,5 V ou d'accumulateurs comparables (13, 14, 15). L'appareil selon l'invention montre pour sa grandeur un maximum de sécurité autonome, dû à la divergence considérable dans la marche des rayons laser en dehors du boîtier et à l'interrupteur-senseur supplémentaire qui se trouve directement dans le domaine de la sortie des rayons.

Ainsi ledit appareil est excellement apte à l'autothérapie dans la médecine humaine et permet à l'utilisateur dans la médecine vétérinaire une application considérablement facilitée.

Comme la production industrielle en masse est facilement possible et les composants ne sont pas chèrs, ledit appareil représente en même temps un appareil laser soft économique pour tout utilisateur.

4. Appareil suivant la revendication 1, caractérisé par le fait qu'une diode lumineuse à faible puissance et/ou un petit transmetteur de signaux acoustiques indiquent l'état de marche de l'appareil.

5. Appareil suivant la revendication 1, caractérisé par le fait que l'amorçage électronique de la diode laser (5, 11) peut comporter un modulateur à fréquence variable pour la gamme de 0,5 à 30 Hz, prévu pour le domaine d'application spéciale de l'acuponcture.

6. Appareil suivant la revendication 1, caractérisé par le fait que la forme de son boîtier permet de l'introduire à un appareil de mesure de puissance et de modulation mobile, et par le fait que lorsque l'appareil pour thérapie à laser (16) est introduit à l'appareil de mesure de puissance et de modulation mobile (22), l'interrupteur (4) de l'appareil pour thérapie à laser (16) est manoeuvré par l'appareil de mesure de puissance et de modulation (22) en raison des formes des appareils conformées l'une à l'autre, et l'appareil pour thérapie à laser (16) se met en marche, un blindage optique de l'appareil de mesure de puissance et de modulation (22) empêchant les rayons diffusés dangereux de sortir pendant le processus de mesure et de réglage dans la région de passage entre la sortie du laser hors du boîtier et le senseur optique (17).

# Fig.1

# Fig.2

# Fig.3

# Fig.4

17

16

18

888 mW      888 Hz      19

20

22

21